# EUROPÄISCHE PATENTSCHRIFT

(11) **EP 1 487 963 B1**
(45) Veröffentlichungstag und Bekanntmachung des Hinweises auf die Patenterteilung: **04.01.2012**
(21) Anmeldenummer: 03744785.1
(22) Anmeldetag: 05.03.2003
(51) Int. Cl.: G01N 33/543

(54) **VORRICHTUNG UND VERFAHREN ZUR DETEKTION VON ZELLULÄREN VORGÄNGEN MITTELS LUMINESZENZMESSUNGEN**
DEVICE AND METHOD FOR DETECTING CELLULAR PROCESSES BY MEANS OF LUMINESCENCE MEASUREMENTS
DISPOSITIF ET PROCEDE DE DETECTION DE PROCESSUS CELLULAIRES PAR DES MESURES DE LUMINESCENCE

(30) Priorität: 27.03.2002 EP 02006978; 26.07.2002 EP 02016793
(43) Veröffentlichungstag der Anmeldung: 22.12.2004
(73) Patentinhaber: Endress+Hauser Conducta Gesellschaft für Mess- und Regeltechnik mbH & Co. KG, 70839 Gerlingen (DE)
(72) Erfinder: KLAPPROTH, Holger, 79108 Freiburg (DE); LEHMANN, Mirko, 79102 Freiburg (DE)
(74) Vertreter: Koslowski, Christine Adelheid
(86) Internationale Anmeldenummer: PCT/EP2003/002252
(87) Internationale Veröffentlichungsnummer: WO 2003/080789

(56) Entgegenhaltungen:
- EP-A- 0 881 490
- EP-A- 1 072 881
- EP-A2- 0 881 490
- EP-A2- 1 072 881
- WO-A-01/13096
- WO-A-01/43875
- WO-A-98/08077
- WO-A-99/57310
- WO-A-03/008974
- US-A- 5 278 048
- US-A- 5 278 048
- US-B1- 6 210 910

## Beschreibung

Die vorliegende Erfindung betrifft eine Vorrichtung nach dem Oberbegriff von Anspruch 1 und ein Verfahren zur Detektion eines Lumineszenzereignisses in, an oder in der unmittelbaren Umgebung einer Zelle, eines Zellverbandes oder eines Gewebes.

Eine derartige Vorrichtung zur optischen Untersuchung einer Zelle ist aus der EP 0 881 490 A2 bekannt. Diese Vorrichtung umfasst ein Substrat, insbesondere ein Halbleitersubstrat, mit einer für die Aufnahme von Zellen geeigneten Oberfläche, einer Anzahl von unterhalb der Oberfläche ausgebildeten Photodetektoren sowie mehreren, oberhalb der Oberfläche angeordneten Lichtquellen. Die matrixartig angeordneten Detektoren erfassen das durch die Lichtquellen ausgesendete Licht, das bedingt durch eine auf die Oberfläche aufgebrachte Zelle an verschiedenen Detektoren unterschiedliche Lichtintensitäten hervorruft, aus welchen eine Information über die Geometrie der Zelle gewonnen werden kann.

Für einige Anwendungen, beispielsweise in der Pharmaforschung, ist es besonders relevant, das Verhalten von Zellen bei einer chemischen oder biochemischen Anregung untersuchen zu können. Vor allem die Untersuchung von Stoffwechselvorgängen an lebenden Zellen ist von besonderem Interesse, da damit z.B. der Einfluss eines neuen potentiellen Medikamentes beobachtet werden kann. Eine häufig durchgeführte Messung ist hierbei die intrazelluläre Kalziumbestimmung mittels kalziumsensitiver Farbstoffe, z.B. Fura II. Aus A.L. Miller, E. Karplus, L.F. Jaffe: "Coeneterate Imaging [Ca2+], with Aequorin Using a Photon Imaging Detector", Meth Cell Biol 40, 305 (1994), ist es bekannt, Zellen, beispielsweise mittels osmotischer Schockbehandlung, mit einem biolumineszenten Stoff, beispielsweise Aqueorin, zu beladen. Intrazelluläre Kalziumsignale als Antwort auf chemische Reize werden dabei durch die Biolumineszenz des Aequorin sichtbar gemacht.

Aus WO 98/08077 A ist ferner eine gattungsfremde Vorrichtung bekannt, die als Trägerelement einen optischen Wellenleiter hat, auf dem eine Sensorschicht angeordnet ist, die mit Rezeptoren versehen ist. Die Rezeptoren binden an einen bestimmten, in einer zu untersuchenden Fluidprobe enthaltenden Analyt. Das Trägerelement bildet eine Innenwand einer Flusszelle, die einen Einlass- und einen Auslasskanal aufweist, durch den die Fluidprobe an der Sensorschicht vorbei zirkulierbar ist. Der Wellenleiter hat ein Einkoppelgitter, das im Abstrahlbereich eines von dem Wellenleiter beabstandeten Halbleiterlasers angeordnet ist. Mit Hilfe des Halbleiterlasers wird eine optische Strahlung erzeugt, die in den Wellenleiter eingekoppelt wird und an deren evaneszentes Feld die Rezeptoren gekoppelt sind. Der Wellenleiter hat außerdem ein von dem Einkoppelgitter beabstandetes Auskoppelgitter, über das die optische Strahlung aus dem Wellenleiter ausgekoppelt und zu einer Fotozelle abgelenkt wird. Nach Angabe der Offenlegungsschrift ist die Vorrichtung insbesondere zur Untersuchung von Flüssigkeiten, wie z.B. Eigelb, Blut, Serum, Plasma oder Urin vorgesehen. Dass an dem Trägerelement Zellen angekoppelt werden können, ist in WO 98/08077 A1 nicht erwähnt.

Aus WO 01/13096 A ist eine gattungsfremde Vorrichtung bekannt, die als Trägerelement einen optischen Wellenleiter aufweist, auf dem zwischen einem im Abstrahlbereich einer Lichtquelle angeordneten Einkoppelgitter und einem einer Photozelle zugewandten Auskoppelgitter ganze Zellen immobilisiert sind. Dabei dienen die Zellen als Erkennungselemente für einen in einer zu untersuchenden Fluidprobe enthaltenden Analyt. Die Vorrichtung ist als geschlossenes FlieBsystem ausgestattet, bei dem die Fluidprobe und gegebenenfalls zusätzliche Reagenzien über Mikrokanäle den Zellen zugeführt werden.

In WO 01/43875 A ist eine Mikrotiterplatte beschrieben, die zwischen einer Grundplatte und einem damit zusammengebrachten Körper mehrere fluidisch gegeneinander abgedichtete Flusszellen aufweist, die jeweils mindestens einen Zulauf und einen Ablauf haben. Der Ablauf jeder Flusszelle führt jeweils zu einem Reservoir, welches aus der Flusszelle austretende Flüssigkeit aufnimmt. In den Flusszellen sind als Erkennungselemente für in zu untersuchenden Fluidproben enthaltene Analyte ganze Zellen oder Zellfragmente aufgebracht.

Aus US 5 278 048 ist eine Vorrichtung bekannt, die eine Flusszelle zur Detektion der Wirkungen von Effektorsubstanzen auf in der Flusszelle vorhandene lebende Zellen aufweist Die Flusszelle hat ein Trägerelement mit einer für das direkte Ankoppeln von Zellen präparierten Oberfläche. In das Trägerelement ist unterhalb der Zellen ist eine Silikon Halbleiter Elektrode integriert. Die Flusszelle hat eine die Oberfläche unter Bildung eines Hohlraumes überdeckende Abdeckung, die eine Zuflussöffnung und eine Abflussöffnung aufweist An die Zuflussöffnung ist eine Anregungsquelle angeschlossen, die ein biologisches oder chemisches Anregungsmedium für die Zellen aufnimmt. Mit der Vorrichtung können jedoch keine Lumineszenzereignisse sichtbar gemacht werden.

In EP 1 072 881 A2 ist ferner eine Vorrichtung mit einer Flusszelle beschrieben, die einen optischen Detektor und einen Hohlraum mit einer Zufluss- und einer Abflussöffnung aufweist Diese Vorrichtung hat jedoch kein Trägerelement mit einer Oberfläche, die für das direkte oder indirekte Ankoppeln von Zellen präpariert ist.

Ziel der vorliegenden Erfindung ist es, eine kompakte und einfach realisierbare Vorrichtung und ein Verfahren der eingangs genannten Art zur Detektion von zellulären Vorgängen mittels Lumineszenzmessungen bei chemischen oder biochemischen Anregungen zu Verfügung zu stellen.

Dieses Ziel wird durch eine Vorrichtung gemäß den Merkmalen des Anspruchs 1 und ein Verfahren gemäß den Merkmalen des Anspruchs 16 gelöst. Vorteilhafte Ausgestaltungen der Erfindung sind Gegenstand der Unteransprüche.

Die Anregungsquelle bildet ein Reservoir für eine chemische oder biologische Substanz, die den Stoffwechsel der Zelle beeinflusst, wobei die Stoffwechselvorgänge durch Chemo- oder Biolumineszenz sichtbar gemacht und mittels des wenigstens einen Detektors detektiert werden.

Unter dem Begriff Chemo- oder Biolumineszenz wird eine durch chemische Anregung hervorgerufene Lumineszenz verstanden. Im Folgenden wird die Chemo- oder Biolumineszenz der Einfachheit halber auch kurz als "Lumineszenz" bezeichnet.

Dieser Prozess unterliegt den allgemeinen Grundsätzen der Quantenmechanik und bewirkt eine Anregung der Atome und Moleküle, die anschließend unter Emission von Licht, welches erfindungsgemäß detektiert wird, in den Grundzustand zurückkehren.

Die erfindungsgemäße Vorrichtung, die zur chemischen Anregung der Zelle zur Detektion der ausgesendeten Lumineszenzsignale dient, umfasst bei einer Ausführungsform ein Wellenlängenfilter zwischen der Oberfläche und dem wenigstens einen Detektor. Vorzugsweise sind eine Vielzahl von Detektoren vorhanden, wobei den einzelnen Detektoren Weltenlängenfilter mit unterschiedlichen Durchlasscharakteristiken zugeordnet sein können, um selektiv Lumineszenzsignale unterschiedlicher Wellenlängen detektieren zu können.

Das Trägerelement ist bei einer Ausführungsform als Halbleiterkörper ausgebildet, wobei eine an die Detektoren angeschlossene Auswerteschaltung vorzugsweise in dem Halbleiterkörper integriert ist. Die Verwendung eines anorganischen. Halbleitermaterials, wie beispielsweise Silizium, besitzt den Vorteil, dass herkömmliche, hinlänglich bekannte Technologieprozesse, beispielsweise CMOS-Prozesse zur Herstellung des Trägers mit den Detektoren und der Auswertschaltung eingesetzt werden können.

Die Integration der Auswerteschaltung in dem Halbleiterchip ermöglicht in unmittelbarer Nähe zu der zu analysierenden Zelle eine Vorverarbeitung der Detektorssignale. Somit handelt es sich bei dieser bevorzugten Ausführungsform der vorliegenden Erfindung um eine "intelligente" Sensoreinrichtung, die wesentlich mehr leistet, als rein passive Sensoren. Beispielsweise können die Ausgangssignale der elektrooptischen Sensoren durch eine mifintegrierte Schaltung so aufbereitet werden, dass sie über Ausgangsschaltungen und Anschlusskontakte relativ problemlos nach außen geführt werden können. Ferner kann die Vorverarbeitung aus der Digitalisierung der analogen Sensorsignale und ihre Umwandlung in einen geeigneten Datenstrom bestehen. Des weiteren kann das signal-to-noise ratio (Signal-RauschVerhältnis) durch die in der erfindungsgemäßen Vorrichtung verwirklichten Nähe des Detektors zum Ort der Signalverarbeitung aufgrund kurzer Signalwege sehr stark verbessert werden. Darüber hinaus sind auch weitere Verarbeitungsschritte möglich, mit denen z.B. die Datenmenge reduziert werden kann oder die der externen Verarbeitung und Darstellung dienen. Damit ist es möglich, dass die verbleibende Auswertung der optischen Signale und ihre Darstellung über einen Personal Computer (PC) erfolgen kann. Ferner kann die erfindungsgemäße Vorrichtung so ausgestaltet sein, dass die vorzugsweise verdichteten bzw. aufbereiteten Daten über eine infrarot- oder Funkverbindung an entsprechend ausgestattete Empfangsstationen übermittelt werden können.

Darüber hinaus besteht auch die insbesondere nach Kostengesichtspunkten interessante Möglichkeit, organische Halbleitermaterialien, die beispielsweise in der EP-A-1 085 319 beschrieben sind, für den Träger mit den Detektoren einzusetzen.

Die Detektoren, die unterhalb der für die Aufnahme von Zellen präparierten Oberfläche angeordnet sind, sind vorzugsweise als Photodioden, CCD-Sensoren oder Photoleiter ausgebildet. Vorzugsweise sind mehrere Detektoren matrixartig in dem Träger integriert, um so eine räumlich aufgelöste Lumineszenzmessung durchführen zu können.

Bei einer Ausführungsform ist vorgesehen, dass die an den Zufluss der Abdeckung angeschlossene Anregungsquelle angesteuert durch die Auswerteschaltung das Anregungsmedium an die Zuflussöffnung, welchem die an der Oberfläche immobilisierte Zelle ausgesetzt wird, abgibt. Zur Steuerung der Medienzufuhr ist beispielsweise ein Ventil in einer Zufuhrleitung zwischen der Anregungsquelle und der Zuflussöffnung angeordnet, das durch die Ansteuerschaltung angesteuert ist.

Um die Zellen an die Oberfläche des Trägerelements anzubinden besteht die Möglichkeit, eine Adhäsionsmatrix oder ein die Zelladhärenz und/oder das Zellwachstum förderndes Medium, beispielsweise Gelatine, auf die Oberfläche aufzubringen und die Zellen bereits auf diesem Nährmedium wachsen zu lassen. Außerdem besteht die Möglichkeit Zellen, auf eine die Zellen immobilisierende Schicht, beispielsweise negativ geladenes Polystyrol, auf die Oberfläche aufzubringen.

Bei einer Ausführungsform der Erfindung ist vorgesehen, dass bereits herstellerseitig eine Zelle oder ein Zellverband an der Oberfläche immobilisiert ist, wodurch es einem Kunden ermöglicht wird unmittelbar Messungen an der Zelle nach Zugabe eines von ihm gewählten Anregungsmediums in die Anregungsquelle durchzuführen.

Vorzugsweise umfasst das Trägerelement mehrere Detektoren oder Detektorenfelder, die räumlich voneinander getrennt sind, um so parallel Messungen an mehreren Zellen oder Zellverbänden durchführen zu können. Diese einzelnen Detektoren oder Detektorfelder sind vorzugsweise derart getrennt angeordnet, dass im Wesentlichen keine Lichtemission eines Punktes oder Feldes von dem oder den Detektoren eines anderen Punktes oder Feldes empfangen werden kann. So können die einzelnen Detektionsorte in jeweiligen Vertiefungen angeordnet sein, wie sie zum Beispiel von üblichen Mikrotiterplatten bekannt sind. Bevorzugt sind erfindungsgemäß muldenartige Vertiefungen und solche, deren seitlichen Wandungen im Wesentlichen senkrecht zur Oberfläche des Sensorchips angeordnet sind. Die jeweiligen Abmessungen einer solchen Vertiefung kann der Fachmann in Kenntnis des Anwendungsbereichs frei wählen, wobei die Vertiefung vorzugsweise um wenigstens 100nm in die Oberfläche des Trägerelements eingesenkt ist.

Alternativ können auf der im wesentlichen planaren Oberfläche senkrecht nach oben gerichtete Trennmittel angeordnet sein, deren Abmessungen vom Fachmann in Kenntnis des gewünschten Anwendungsbereiches und der räumlichen Abmessung der Zellen ausgewählt werden können. Die Anbringung entsprechend geeigneter Trennmittel kann beispielsweise durch anodisches Bonden oder durch sogenannte Flip-Chip- Verfahren erfolgen. Eine solche Vorrichtung mit mehreren Detektorfeldern ermöglicht erfindungsgemäß ein sensorgestütztes elektrooptisches Bildaufnahmeverfahren.

Bei dem erfindungsgemäßen Verfahren wird an der für die Aufnahme von Zellen präparierten Oberfläche wenigstens eine Zelle immobilisiert, wobei anschließend ein physikalisches oder chemisches Anregungsmedium aus der Anregungsquelle in den Hohlraum zugeführt und daraus resultierende Lumineszenzereignisse mittels des wenigstens einen Detektors erfasst werden.

Die Lumineszenzereignisse werden vorzugsweise zeitlich aufgelöst erfasst. Das heißt nach der Abgabe eines Anregungsmediums an die Umgebung der Zelle, werden die Lichtverhältnisse an den Detektoren zu mehreren aufeinanderfolgenden Zeitpunkten, beispielsweise im Takt von, ins ausgewertet.

Um Stoffwechselvorgänge in der Zelle mittels Lumineszenz sichtbar zu machen, ist der Einsatz von Luminophoren erforderlich, die auf die interessierenden Stoffwechselprodukte mit Lumineszenz reagieren, die durch die Detektoren erfasst wird.

Für den jeweiligen Anwendungsfall geeignete Luminophore können von einem Fachmann werden abhängig von dem zu detektierenden Stoffwechselprodukt, mit welchem die Luminophore reagieren sollen, in hinlänglich bekannter Weise ausgewählt werden.

Man kann Luminophore mit unterschiedlichen Halbwertzeiten unterscheiden. Die Halbwertzeit gibt an, wie lange Lumineszenz nach einer Anregung messbar ist. Die Auswahl eines Luminophors mit einer für den jeweiligen Anwendungsfall geeigneten Halbwertzeit liegt im Ermessen des Fachmanns. Besonders geeignet sind insbesondere Luminophore, deren Halbwertzeit deutlich größer als 5ns, vorzugsweise zwischen 100µs und 2000µs, liegt.

Grundsätzlich geeignet sind organische Luminophore, Selten Erden Metalle (SEE) bzw. Lanthaniden oder Actinidenverbindungen, "Microspheres" (z.B. FluoSpheres^{®} Europium Luminescent Microspheres, Molecular Probes) oder Nanokristalle von Halbleitern, wobei letztere neben ihren Lumineszenzeigenschaften insbesondere eine relativ kleine Größe (wenige nm) und eine hohe Stabilität (kein Photobleaching) aufweisen. Weitere geeignete Luminophore sind Erdalkalihalogenide mit Gitterfehlstellen, wie sie z.B. durch Dotierungen (Fremdionen) oder radioaktive Strahlung hergestellt werden können.

Die meisten vorgenanten Farbstoffe sind nur für die Messung an oder in der unmittelbaren Umgebung der Zellen geeignet. Allerdings gibt es Verfahren um Zellmembranen für Farbstoffe und Reportermoleküle permeabel zu machen. Derartige Verfahren sind z.B.:
- Acetoxymethyl (AM) ester loading
- Acid loading (insbesondere für Pfanzenzellen)
- ATP-induzierte Ppermeabilisierung
- Cationic liposome delivery
- Electroporation
- Hypoosmotischer Shock
- Influx pinocytic cell-loading reagent

Ein geeignetes Beladesystem ist z.B. in K. Barber, et al.: "Delivery of Membrane-Impermeant Fluorescent Probes into Living Neural Cell Populations by Lipotransfer.", Neurosci Lett 15 207, 17 (1996) beschrieben.

Bei einer Ausführungsform des Verfahrens ist vorgesehen, die durch den oder die Detektor(en) detektierten Lumineszenzsignals mit einem Referenzwert zu vergleichen. Dieser Referenzwert kann in einem Speicher in dem Halbleiterkörper gespeichert werden und kann beispielsweise durch eine Messung vor der Anregung der Zelle erhalten werden. Der Referenzwert kann auch parallel zu der Messung an der Zelle erhalten werden, indem mehrere räumlich getrennte Detektoren oder Detekorfelder bereitgestellt werden, wobei im Bereich eines Detektors oder Detektorfeldes keine Zelle immobilisiert ist, so dass detektierte Lumineszenzsignale dieses Detektors oder Detektorfeldes als Referenzwert verwendet wird, der Licht bzw. Strahlungseinflüsse berücksichtigt, die nicht durch die zu analysierende Zelle bedingt sind, wie z.B. die Eigenfluoreszenz von Systemkomponenten, und die somit herausgerechnet werden können.

Sofern die Sensoroberfläche das Design einer Microarraynordnung aufweist, bei der eine Vielzahl von Detektorfeldern mit jeweils einer Anzahl von Detektoren vorhanden sind, kann die Detektion der Messfeld- bzw -punktsignalwerte sequentiell erfolgen, indem z.B. ganze Zeilen oder Spalten der Sensoroberfläche bzw Teile derselben nacheinander detektiert werden (Multiplexanwendung).

Die Verwendung mehrerer paralleler Detektorfelder, an denen jeweils dieselben Messungen durchgeführt werden, bietet darüber hinaus den Vorteil, dass mehrere Messergebnisse vorliegen, aus denen das Gesamt-Messergebnis gemittelt werden kann.

Die Ausgangssignale der Detektoren können in dem Halbleiterchip ausgewertet oder mittels geeigneter Schaltungseinrichtungen nach einer Analog-Digitalumsetzung einer externen Auswerteeinrichtung zugeführt werden.

Für den Fachmann ist klar, dass die Wahl des Detektors bzw. des Materials von der zu detektierenden Emissionswellenlänge des Farbstoffes abhängt. Grundsätzlich besitzt der Detektor aufgrund des sogenannten "Halbleiterbandgaps" je nach Materialwahl (z.B. Silizium oder Germanium) unterschiedliche Empfindlichkeiten bezüglich Wellenlänge der detektierten Lumineszenzsignals. Im bevorzugten Falle der Verwendung einer Silizium-Photodiode wird ein Empfindlichkeitsbereich geschaffen, der vom infraroten bis in das ultraviolette Wellenspektrum reicht, wobei die Empfindlichkeit zwischen diesen Bereichen am größten. (s. z.B. B. Streetman: "Solid State Electronic Devices", , Prentice-Hall, Inc., ISBN 0-13-10 436379-5, S. 201-227 (1995).

Um Lumineszenzen unterschiedlicher Wellenlängen detektieren zu können werden entweder wellenlängenspezifische Photoelemente oder aber herkömmliche Photodioden ausgewählt, die mit aufgelegten, aufgebrachten, aufgedampften oder integrierten Wellenlängenfiltern ausgestattet sind. So ist z.B. bekannt, dass Siliziumnitrid im Gegensatz zu Siliziumoxid UV-Licht nicht durchläst, und dass Polysilizium UV-Strahlung absorbiert (s. z.B. v.P. Iordanov et al.: "Integrated high rejection filter for NADH fluorescence measurements", Sensors 2001 Proceedings, Vol. 1,8 -10 Mai, S. 106- 111, AMA-Service (2001)). Daher kann auf die Gateoxidschicht im Rahmen des üblichen CMOS-Prozesses Nitrid oder Polysilizium deponiert werden, wodurch auf der Photodiode entsprechende Filter geschaffen werden. So hat z.B. NADH (Nicotinamid Adenine Dinucleotid) eine Anregungswellenlänge von 350 nm und eine Emissionswellenlänge von 450 nm. Durch Aufbringung eines Filters, der 350nm ausfiltert, kann daher die Sensitivität erhöht werden.

Dieser Effekt kann genutzt werden, um bei paralleler Verwendung von z.B. zwei unterschiedlichen Luminophoren, von denen beispielsweise nur einer Licht im UV-Bereich emittiert, eine differentielle Detektion zu ermöglichen, da die hierfür vorgesehenen Detektoren UV-sensitiv ausgestaltet sind oder nicht. Ferner bietet dieser Effekt die Möglichkeit, gegebenenfalls störende Eigenfluoreszenzen anwesender Materialien mit bekannter Emissionswellenlänge durch Bereitstellung entsprechender Filter aus dem Messverfahren herauszunehmen. Ein Beispiel hierfür ist die parallele Verwendung von Europium-Chelaten (Emission bei ca. 620 nm) und mit Kupfer dotiertem Zinksulfid (Emission bei ca. 525 nm), die durch hinreichend voneinander verschiedenen Emissionswellenlängenbereichen eine Zweifarbdetektion ermöglichen, z.B. innerhalb eines Bereichs eines Detektorpunktes bzw .-feldes, indem z.B. die eine Hälfte der Sensoren eines Detektorpunktes bzw. -feldes mit einem Tiefpassfilter und die andere Hälfte der Sensoren des gleichen Punktes oder Feldes mit einem Hochpassfilter ausgestattet ist.

Zusätzlich oder alternativ können unterschiedliche Luminophoren parallel eingesetzt werden, sofern ihre physikalischen bzw. optischen Eigenschaften hinreichend voneinander abweichen. Beispielsweise werden erfindungsgemäß die unterschiedlichen Anregungswellenlängen zweier zu verwendender Luminophore A und B und/oder deren unterschiedlichen Halbwertzeiten genutzt. Dies kann z.B. durch Bereitstellung von zwei unterschiedlich dotierten Nanokristallen erfolgen.

Die Signale der Detektoren werden durch eine Auswertungseinheit aufgenommen. Die Auswertungseinheit besitzt einen sehr schnellen Konverter zur Umwandlung analoger Detektorsignale in digitale Werte, die gespeichert werden. Eine Auswertung der digitalen Werte wird vorzugsweise in Echtzeit vorgenommen, kann jedoch auch zeitlich verzögert erfolgen. Zur Auswertung der digitalen Werte kann ein gewöhnlicher Mikroprozessor verwendet werden. Für den Fall, dass das Lumineszenzsignal für eine eindeutige Detektion zu schwach ist, kann im Rahmen einer bevorzugten Ausführungsform der Detektion eine Erhöhung der Nachweissensitivität über eine Integration mehrerer Einzelmessungen erreicht werden. Dabei erfolgt eine identische Messung mehrfach und die Messergebnisse werden aufaddiert. Dies kann sowohl direkt auf dem Sensorchip als auch nach der Messung über geeignete Software erfolgen.

Die vorliegende Erfindung wird nachfolgend in Ausführungsbeispielen anhand von Figuren näher erläutert. In den Figuren zeigt:
- Figur 1: ein erstes Ausführungsbeispiel einer erfindungsgemäßen Vorrichtung zur Detektion von zellulären Vorgängen mittels Lumineszenzmessungen in Seitensicht im Querschnitt,
- Figur 2: ein zweites Ausführungsbeispiel einer erfindungsgemäßen Vorrichtung in Seitenansicht im Querschnitt,
- Figur 3: ein drittes Ausführungsbeispiel einer erfindungsgemäßen Vorrichtung in Seitenansicht im Querschnitt,
- Figur 4: ein viertes Ausführungsbeispiel einer erfindungsgemäßen Vorrichtung in Seitenansicht im Querschnitt.

In den Figuren bezeichnen sofern nicht anders angegeben gleiche Bezugszeichen gleiche Teile mit gleicher Bedeutung.

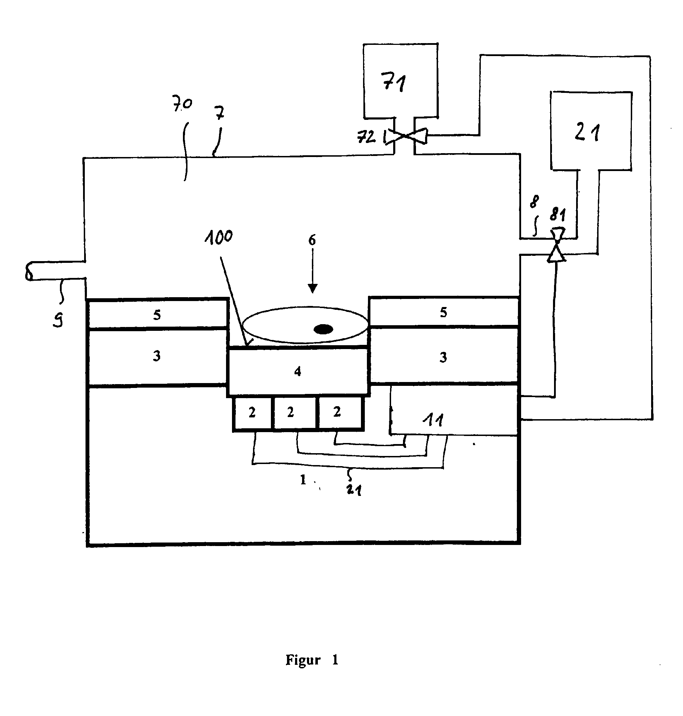

Figur 1 zeigt ein erstes Ausführungsbeispiel einer erfindungsgemäßen Vorrichtung zur Detektion eines Lumineszenzereignisses in, an oder in der unmittelbaren Umgebung einer Zelle, eines Zellverbandes oder eines Gewebes, wobei in Figur 1 zu Zwecken der Veranschaulichung lediglich eine Zelle 6 dargestellt ist. Die Vorrichtung umfasst ein Trägerelement 1 mit einer für das direkte oder indirekte Ankoppeln von Zellen präparierten Oberfläche 100. Hierzu umfasst die Oberfläche beispielsweise eine Gelatineschicht, auf der die Zelle bereits gewachsen ist, oder eine andere für die Immobilisierung einer Zelle oder eines Zellverbandes geeignete Substanz. Oberhalb der Oberfläche 100 ist eine einen Hohlraum 70 bildende Abdeckung vorhanden, wobei die Zelle in diesem Hohlraum angeordnet ist. Die Abdeckung umfasst einen Zufluss 8 und einen Abfluss 9, wobei der Zufluss 8 an eine ein Anregungsmedium aufnehmende Anregungsquelle 21 angeschlossen ist.

Die Vorrichtung dient zur Untersuchung des Verhaltens der Zelle 6 bei einer chemischen oder biochemischen Anregung durch das in der Anregungsquelle 21 enthaltene Anregungsmedium mittels Lumineszenzmessungen. Die Lumineszenz wird durch Luminophore erzeugt, die in hinlänglicher bekannter Weise in die Zelle oder in eine Nährlösung in der Umgebung der Zelle eingebracht sind, und die beispielsweise mit einem Stoffwechselprodukt der Zelle reagieren. Die Lumineszenz wird durch Detektoren 2, beispielsweise Photodioden detektiert, die unterhalb der Oberfläche 100 in dem Trägerelement integriert sind. In dem Beispiel ist eine Wellenlängenfilter 4 zwischen der Oberfläche 100 mit der Zelle 6 und den Detektoren 2 ausgebildet, wobei auf den Detektoren 6 vorzugsweise eine nicht näher dargestellte optisch transparente Isolationsschicht aufgebracht ist, die Leckströme über die Oberfläche 100 verhindert, sofern das Filter 4 nicht elektrisch isolierend ist. zusätzlich mit einem Filter 4 versehen sein.

Auf den Träger 1 ist in dem Beispiel ein Kratzschutz 3 mit einer Oberflächenbeschichtung 5, beispielsweise einem Edelmetall oder einem hydrophoben/hydrophilen Material, aufgebracht, wobei dieser Kratzschutz 3 oberhalb der Detektoren eine Aussparung aufweist, am Boden derer das Filter 4 und die für die Aufnahme der Zelle präparierte Oberfläche 100 vorhanden ist.

In dem Trägerelement, beispielsweise einem Halbleiterchip, ist in dem Beispiel weiterhin eine - in der Figur 1 schematisch dargestellte - Auswerteschaltung 11 integriert, die an die Detektoren 2 angeschlossen ist. Leitungsverbindungen 21 zwischen der Auswerteschaltung 11 und den Detektoren 2 sind in Figur 1 lediglich schematisch dargestellt.

Dem Hohlraum 70 ist aus einem Flüssigkeitsreservoir 71 eine zum Aufbewahren bzw. Nasshalten oder auch Waschen der Zelle geeignete Flüssigkeit zuführbar, beispielsweise eine Nährlösung. Zur Anregung der Zelle wird dieser Flüssigkeit das Anregungsmedium aus der Anregungsquelle zugeführt, wobei die Zufuhr des Anregungsmediums in dem Beispiel mittels eines in der Zuführleitung 8 angeordneten Ventils 81 steuerbar ist. Die Steuerung dieses Ventils 81 erfolgt in dem Beispiel durch die integrierte Auswerteschaltung 11.

Das Anregungsmedium ist beispielsweise so gewählt, dass es den Stoffwechsel der Zelle beeinflusst, wobei Stoffwechselvorgänge durch die erläuterten Luminophore sichtbar und durch die Detektoren 2 detektiert werden. Vorzugsweise sind mehrere Anregungsquellen 21 vorhanden, die unabhängig voneinander ein Anregungsmedium abgeben, um so zeitlich aufeinanderfolgend das Zellenverhalten bei verschiedenen Anregungsmedien untersuchen zu können.

Um eine die Zelle 6 anregende chemische Substanz nach Ende der Untersuchung abzuführen wird beispielsweise mit der Flüssigkeit aus dem Reservoir 71 gespült, wodurch das Medium über den Abfluss 9 abfließt, oder die Flüssigkeit wird abgesaugt. Die Flüssigkeitszufuhr aus diesem Reservoir wird vorzugsweise ebenfalls über ein Ventil 72 gesteuert, das durch die Auswerteschaltung angesteuert ist.

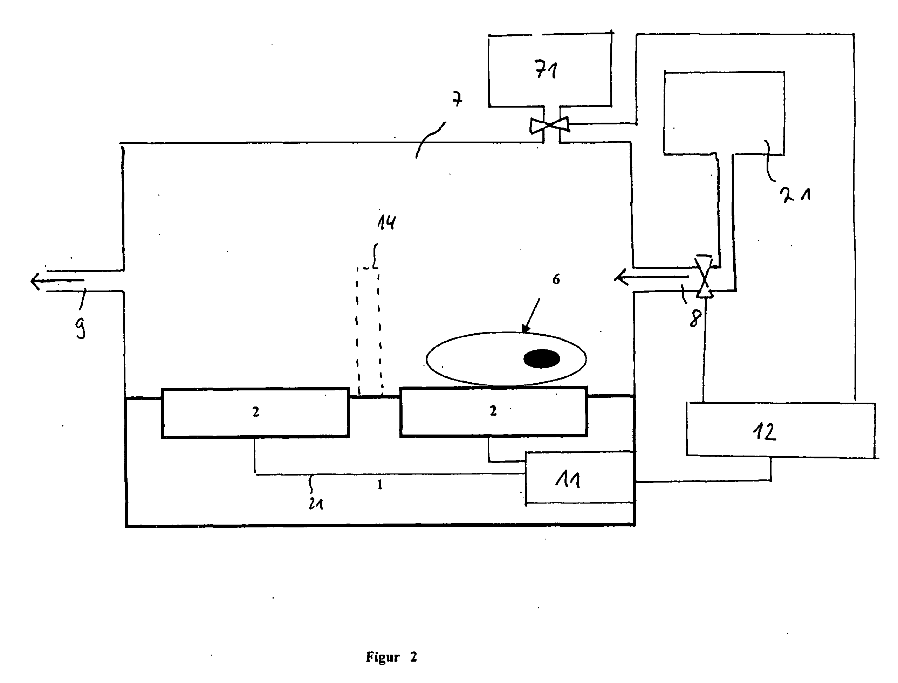

Die Steuerung der Ventile kann auch mittels einer externen Ansteuerschaltung 12 erfolgen, der auch ein von den Ausgangssignalen der Detektoren 2 abhängiges Signal der Auswerteschaltung 11 zugeführt sein kann, wie dies in Figur 2 dargestellt ist.

Figur 2 zeigt ein zweites Ausführungsbeispiel einer erfindungsgemäßen Vorrichtung, bei der zwei räumlich voneinander getrennte Detektoren 2 unterhalb einer für die Aufnahme von Zellen 6 geeigneten Oberfläche in einem Träger 1, beispielsweise einem, Halbleiterkörper 1 integriert sind. Dabei ist nur oberhalb eines der beiden Detektoren 2 eine Zelle 6 immobilisiert. Der andere Detektor dient zur Bereitstellung eines Referenzwertes für den durch den Detektor 2 unterhalb der Zelle 6 gelieferten Messwert. Der Referenzwert berücksichtigt dabei gegebenenfalls unabhängig von einer Anregung vorhandene, beispielsweise aus einer Eigenfluoreszenz der Systemkomponenten resultierende Lumineszenzsignale, die aus dem Messergebnis herauszurechnen sind. Wie gestrichelt dargestellt ist, ist vorzugsweise eine senkrecht von dem Träger 1 aufragende Trennwand 14 vorhanden, die verhindert, dass der den Referenzwert erzeugende Detektor 2 durch spezifische Lumineszenzsignale aus der Zelle 6 oder der Umgebung der Zelle beeinflusst wird.

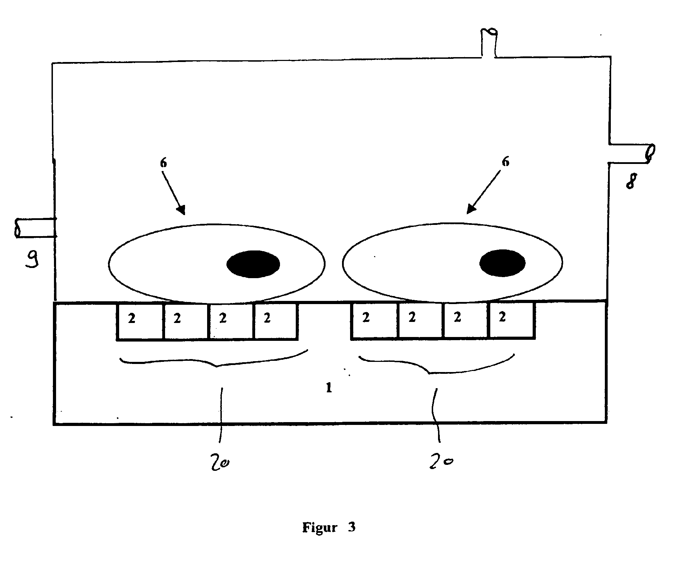

Figur 3 zeigt ein Ausführungsbeispiel einer erfindungsgemäßen Vorrichtung mit einer Trägeranordnung, die sich bezüglich der Anordnung der Detektoren 2 von den beiden vorherigen Ausführungsbeispielen unterscheidet. Auf die Darstellung der Anregungsquelle und der Auswerteschaltung die selbstverständlich auch vorhanden sind, ist bei dem Ausführungsbeispiel gemäß Figur 3 verzichtet.

Der vorzugsweise aus einem Halbleiterchip gebildete Träger umfasst in dem Beispiel zwei Detektorfelder 20, die jeweils mehrere Detektoren umfassen. Hierdurch können mehrere Messungen gleichzeitig durchgeführt werden, wodurch eine statistische Abschätzung des spezifischen erhaltenen Messsignals abgeleitet werden kann. Beispielsweise wird hierdurch ermöglicht, dass zwischen unspezifischen und spezifischen Signalen differenziert werden kann, wobei spezifische Signale die aus Stoffwechselvorgängen in der Zelle resultierenden Signal und unspezifische Signale andere Signale, beispielsweise Störsignale, sind. Diese unspezifischen Signale besitzen eine andere Verteilung als die spezifischen Signale, so dass eine Unterscheidung möglich wird.

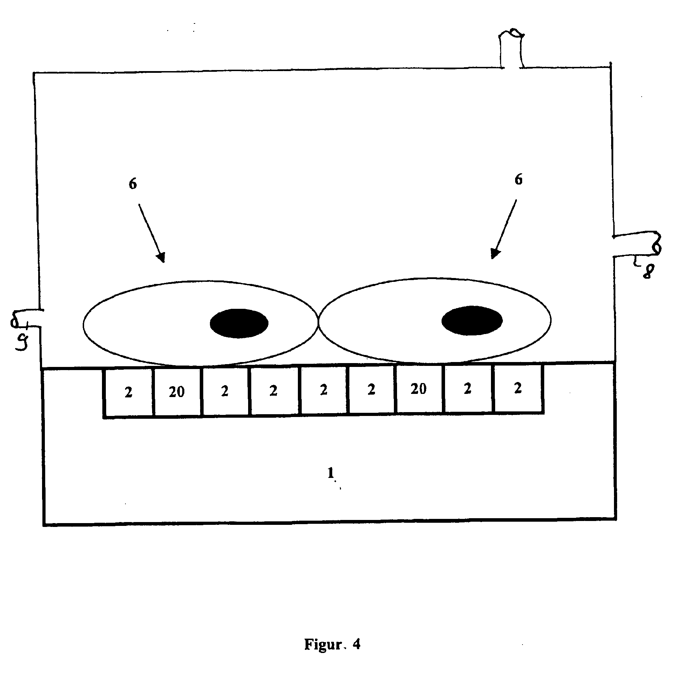

Figur 4 zeigt eine Abwandlung eines in Figur 3 dargestellten Trägerelements 2, das ein zusammenhängendes Detektorfeld 20 mit einer Vielzahl von Detektoren 2 aufweist, welches dazu geeignet ist mehrere Zellen gleichzeitig zu untersuchen. Diese Vorrichtung ermöglicht es, identische Zellen unter vergleichbaren Bedingungen zu untersuchen und deren Signale zu detektieren, wobei diese unter gleichen Bedingungen gewonnenen Signale zur statistischen Absicherung in einer Auswerteeinheit gemittelt werden können.

Ein Verfahren zur Herstellung eines für die erfindungsgemäße Vorrichtung geeigneten Trägers wird nachfolgend kurz erläutert:

Ein Halbleiterchip wird unter Verwendung von 6" (Inch) Wafern mit einem 0.5 µm CMOS-Prozess gefertigt. Die Detektoren 2 werden als pn-Photodioden in einer n-Wanne auf einem p-Substrat angeordnet. Nach der Feldoxidation folgen die Definition der p-Gebiete der Photodiode und die Aufbringung einer 10 nm dicken Gateoxidschicht. Gewünschtenfalls kann an dieser Stelle zusätzlich noch ein strukturiertes Nitrid (z.B. LPCVD oder PECVD) als UV -Filter aufgebracht werden. Dann erfolgt die Auflagerung und Strukturierung einer Siliziumdioxid-Schicht. Anschließend werden die weiteren üblichen CMOS-Schritte durchgeführt, wie z.B. das Aufbringen einer Verdrahtungsschicht und die Oberflächenpassivierung (Kratzschutz).

Der so hergestellte CMOS-Sensor wird durch Beschichten mit einem geeigneten Zellwachstumssubstrat, z.B. Gelatine, modifiziert. Auf dieses Substrat werden dann vereinzelte Zellen (z.B. trypsinisierte Epithelzellen) ausgesät und mit Zellkulturmedium (z.B. DMEM-FI2) zum Wachsen gebracht.

Die erfindungsgemäße Vorrichtung eignet sich beispielsweise zur Untersuchung des Kalziumstoffwechsels von Herzmuskelzellen. Dabei werden die Zellen zunächst durch osmotische Schockbehandlung mit Aqueorin beladen. Intrazelluläre Kalziumsignale als Antwort auf chemische Reize durch die aus der Anregungsquelle 21 abgegebene chemische Substanz werden durch die Biolumineszenz des Aqueorins sichtbar und durch die Detektoren detektiert.

Die erfindungsgemäße Vorrichtung zur Untersuchung intrazellulärer Vorgänge nach einer biologischen oder chemischen Anregung ist besonders kompakt und mittels bekannter Verfahren herstellbar.

## Patentansprüche

1. Vorrichtung zur Detektion eines Lumineszenzereignisses in, an oder in der unmittelbaren Umgebung einer Zelle, eines Zellverbandes oder eines Gewebes, die folgende Merkmale aufweist
(a) ein Trägerelement (1) mit einer für das direkte oder indirekte Ankoppeln von Zellen präparierten Oberfläche (100),
(b) wenigstens einen optischen Detektor (2), der in dem Trägerelement (1) unterhalb der Oberfläche (100) integriert ist,
**gekennzeichnet durch** folgende Merkmale:
(c) eine die Oberfläche (100) unter Bildung eines Hohlraumes (70) überdeckende Abdeckung (7), die eine Zuflussöffnung (8) und eine Abflussöffnung (9) aufweist,
(d) eine an die Zuflussöffnung (8) angeschlossene Anregungsquelle (21), die ein biologisches oder chemisches Anregungsmedium für Zellen aufnimmt,
(e) der Detektor (2) ist zum Empfangen eines Chemo- oder Biolumineszenzsignals ausgestaltet.

2. Vorrichtung nach Anspruch 1, bei der ein Filter (4) zwischen der Oberfläche (100) und dem wenigstens einen Detektor (2) ausgebildet ist.

3. Vorrichtung nach Anspruch 1 oder 2, bei der das Trägerelement (1) ein Halbleiterkörper ist.

4. Vorrichtung nach einem der vorangehenden Ansprüche, bei der mehrere Detektoren (2) unterhalb der für das Ankoppeln der Zellen präparierten Oberfläche (100) in dem Trägerelement (1) integriert sind.

5. Vorrichtung nach Anspruch 4, bei der der wenigstens eine Detektor (2) eine Photodiode ist.

6. Vorrichtung nach einem der vorangehenden Ansprüche, bei der eine an den wenigstens einen Detektor (2) angeschlossene Auswerteschaltung (11) vorgesehen ist.

7. Vorrichtung nach Anspruch 6, bei der die Auswerteschaltung (11) in dem Trägerelement (1) integriert ist.

8. Vorrichtung nach Anspruch 6 oder 7, bei der die Anregungsquelle (21) angesteuert durch die Auswerteschaltung eine chemische oder biologische Substanz an die Zuflussöffnung (8) abgibt

9. Vorrichtung nach Anspruch 8, bei der zur Steuerung der Medienzufuhr ein Ventil in einer Zuführleitung zwischen der Anregungsquelle (21) und der Zuflussöffnung (8) angeordnet ist.

10. Vorrichtung nach einem der vorangehenden Ansprüche, bei der eine Adhäsionsmatrix und/oder ein Wachstumssubstrat für Zellen auf die Oberfläche (100) aufgebracht ist.

11. Vorrichtung nach Anspruch 10, bei der das Wachstumssubstrat Gelatine ist.

12. Vorrichtung nach einem der Ansprüche 1 bis 9, bei der ein Zellen immobilsierendes Medium auf die Oberfläche (100) aufgebracht ist.

13. Vorrichtung nach Anspruch 12, bei der das Medium Polystyrol, vorzugsweise negativ geladenes Polystyrol ist.

14. Vorrichtung nach einem der vorangehenden Ansprüche, bei der wenigstens eine Zelle (6) an der Oberfläche immobilisiert ist.

15. Vorrichtung nach einem der vorangehenden Ansprüche, bei der die für die Aufnahme von Zellen präparierte Oberfläche der Trögervonichtung gegenüber Oberflächenbereichen (101), die nicht für die Aufnahme von Zellen präpariert sind, vertieft ausgebildet ist, wobei diese Vertiefung vorzugsweise wenigstens 100nm beträgt.

16. Verfahren zur Detektion eines Lumineszenzereignisses in, an oder in unmittelbarer Umgebung einer Zelle, eines Zellverbandes oder eines Gewebes, das folgende Merkmale aufweist
- Bereitstellen einer Sensorvorrichtung nach einem der Ansprüche 1 bis 15,
- Immobilisieren der Zelle an der für die Aufnahme von Zellen präparierten Oberfläche (100),
- Einbringen eines mit einem Stoffwechselprodukt der Zelle reagierenden Luminophors in die Zelle (6) oder in die Umgebung der Zelle,
- Anregen der Zelle mittels einer chemischen oder biologischen Substanz, die Stoffwechselvorgänge der Zelle durch Chemo- oder Biolumineszenz sichtbar macht,
- Detektieren eines Chemo- oder Biolumineszenzsignals.

17. Verfahren nach Anspruch 16, bei dem das Lumineszenzsignal zeitlich aufgelöst detektiert wird.

## Claims

1. A device for detecting a luminescence event in, at, or in the immediate vicinity of a cell, a cell cluster or a tissue, the device featuring the following:
(a) a carrier element (1) with a surface (100) prepared for direct or indirect coupling of cells;
(b) at least one optical detector (2) integrated into the carrier element (1) below the surface (100);
**characterized by** the following features:
(c) a cover (7) covering the surface (100) to form a cavity (70), said cover having an inlet opening (8) and an outlet opening (9);
(d) an excitation source (21) connected to the inlet opening (8), the excitation source holding a biological or chemical excitation medium for cells;
(e) the detector (2) is designed to receive a chemical or biological luminescence signal.

2. The device as claimed in Claim 1 where a filter (4) is located between the surface (100) and the one detector (2) at least.

3. The device as claimed in Claim 1 or 2 where the carrier element (1) is a semiconductor body.

4. The device as claimed in one of the previous claims, where multiple detectors (2) are integrated into the carrier element (1) below the surface (100) prepared for the coupling of cells.

5. The device as claimed in Claim 4, where the one detector (2) at least is a photodiode.

6. The device as claimed in one of the previous claims, where an evaluation circuit (11) is connected to the one detector (2) at least.

7. The device as claimed in Claim 6, where the evaluation circuit (11) is integrated into the carrier element (1).

8. The device as claimed in Claim 6 or 7, where the excitation source (21) - controlled by the evaluation circuit - sends a chemical or biological substance to the inlet opening (8).

9. The device as claimed in Claim 8, where a valve is arranged in an inlet line between the excitation source (21) and the inlet opening (8) to control the supply of medium.

10. The device as claimed in one of the previous claims, where an adhesion matrix and/or a growth substrate for cells is applied to the surface (100).

11. The device as claimed in Claim 10, where the growth substrate comprises gelatin.

12. The device as claimed in one of the Claims 1-9, where a cell-immobilizing medium is applied to the surface (100).

13. The device as claimed in Claim 12, where the medium is polystyrene, preferably negatively charged polystyrene.

14. The device as claimed in one of the previous claims, where at least one cell (6) is immobilized at the surface.

15. The device as claimed in one of the previous claims, where the surface of the carrier unit prepared to receive cells has a depression relative to the parts of the surface (101) that are not prepared to receive cells, said depression preferably being at least 100nm.

16. A method for detecting a luminescence event in, at, or in the immediate vicinity of a cell, a cell cluster or a tissue, the method featuring the following:
- The provision of a sensor device as claimed in one of the Claims 1-15;
- The immobilization of the cell at the surface (100) prepared to receive cells;
- The introduction of a luminophore into the cell (6) or the vicinity of the cell, said luminophore reacting with a metabolic product of the cell;
- The excitation of the cell using a chemical or biological substance which makes the metabolic processes of the cell visible by chemical or biological luminescence;
- The detection of a chemical or biological luminescence signal.

17. Method as claimed in Claim 16, where the luminescence signal is detected in a time-resolved manner.

## Revendications

1. Procédé destiné à la détection d'un événement de luminescence dans, sur ou à proximité immédiate d'une cellule, d'un amas de cellules ou d'un tissu, qui présente les caractéristiques suivantes :
(a) un élément support (1) ayant une surface (100) préparée pour le couplage direct ou indirect de cellules et (b),
(b) au moins un détecteur optique (2), qui est intégré à l'élément support (1) sous la surface (100),
**caractérisé par** les caractéristiques suivantes:
(c) un couvercle (7) pourvu d'un orifice d'entrée (8) et d'un orifice de sortie (9) recouvre la surface (100) en formant une cavité (70),
(d) une source d'excitation (21) est reliée à l'orifice d'entrée (8), laquelle source contient un produit d'excitation biologique ou chimique pour cellules,
(e) le détecteur (2) est conçu pour la réception d'un signal de chimioluminescence ou de bioluminescence.

2. Dispositif selon la revendication 1, pour lequel un filtre (4) est formé entre la surface (100) et l'au moins un détecteur (2).

3. Dispositif selon la revendication 1 ou 2, pour lequel l'élément support (1) est un corps semi-conducteur.

4. Dispositif selon l'une des revendications précédentes, pour lequel plusieurs détecteurs (2) sont intégrés dans l'élément support (1), sous la surface (100) préparée pour le couplage des cellules.

5. Dispositif selon la revendication 4, pour lequel l'au moins un détecteur (2) est une photodiode.

6. Dispositif selon l'une des revendications précédentes, pour lequel est prévu un circuit d'exploitation (11) connecté à l'au moins un détecteur (2).

7. Dispositif selon la revendication 6, pour lequel le circuit d'exploitation (11) est intégré dans l'élément support (1).

8. Dispositif selon la revendication 6 ou 7, pour lequel la source d'excitation (21), commandée par le circuit d'exploitation, délivre une substance chimique ou biologique à l'orifice d'entrée (8).

9. Dispositif selon la revendication 8, pour lequel est disposée une vanne, en vue de la commande de l'alimentation de produit, dans une conduite d'alimentation entre la source d'excitation (21) et l'orifice d'entrée (5).

10. Dispositif selon l'une des revendications précédentes, pour lequel est appliquée sur la surface (100) une matrice d'adhésion et/ou un substrat de croissance pour cellules.

11. Dispositif selon la revendication 10, pour lequel le substrat de croissance est de la gélatine.

12. Dispositif selon l'une des revendications 1 à 9, pour lequel est appliqué sur la surface (100) un produit immobilisant les cellules.

13. Dispositif selon la revendication 12, pour lequel le produit est du polystyrène, de préférence du polystyrène chargé négativement.

14. Dispositif selon l'une des revendications précédentes, pour lequel au moins une cellule (6) est immobilisée sur la surface.

15. Dispositif selon l'une des revendications précédentes, pour lequel la surface du dispositif support, préparée pour la réception de cellules, est plus profonde que les zones de surface (101), qui ne sont pas préparées pour la réception de cellules, cette profondeur supplémentaire s'élevant de préférence à au moins 100 nm.

16. Procédé destiné à la détection d'un événement de luminescence dans, sur ou à proximité immédiate d'une cellule, d'un amas de cellules ou d'un tissu, qui présente les caractéristiques suivantes :
- mise à disposition d'un dispositif capteur selon l'une des revendications 1 à 15,
- immobilisation de la cellule sur la surface (100) préparée sur la réception de cellules,
- incorporation dans la cellule (6) ou dans l'environnement de la cellule d'un luminophore réagissant avec un métabolite de la cellule,
- excitation de la cellule au moyen d'une substance chimique ou biologique, qui rend visible les processus métaboliques de la cellule par chimioluminescence ou bioluminescence.
- détection d'un signal de chimioluminescence ou de bioluminescence.

17. Procédé selon la revendication 16, pour lequel le signal de luminescence est détecté par résolution temporelle.
